(19) Europäisches Patentamt
European Patent Office
Office européen des brevets

(11) Veröffentlichungsnummer : **0 385 194 B1**

# (12) EUROPÄISCHE PATENTSCHRIFT

(45) Veröffentlichungstag der Patentschrift : **17.06.92 Patentblatt 92/25**

(21) Anmeldenummer : **90102985.0**

(22) Anmeldetag : **16.02.90**

(51) Int. Cl.$^5$ : **C07D 493/10,** // (C07D493/10, 319:00, 317:00), (C07D493/10, 321:00, 319:00), (C07D493/10, 321:00, 317:00), (C07D493/10, 317:00, 317:00)

## (54) Verfahren zur Herstellung von unsymmetrischen Spiroorthocarbonaten.

(30) Priorität : **01.03.89 DE 3906464**

(43) Veröffentlichungstag der Anmeldung : **05.09.90 Patentblatt 90/36**

(45) Bekanntmachung des Hinweises auf die Patenterteilung : **17.06.92 Patentblatt 92/25**

(84) Benannte Vertragsstaaten : **DE FR GB IT**

(56) Entgegenhaltungen :
**DE-A- 3 225 818**
**DE-A- 3 531 454**
**SYNTHESIS, Oktober 1984, Seite 837; TAKESHI E. et al.: "A facile synthesis of spiro orthocarbonates"**

(73) Patentinhaber : **BAYER AG**
**W-5090 Leverkusen 1 Bayerwerk (DE)**

(72) Erfinder : **Mues, Peter, Dr.**
**Weiers Hecke 30**
**W-4100 Duisburg 46 (DE)**
Erfinder : **Buysch, Hans-Josef, Dr.**
**Brandenburgerstrasse 28**
**W-4150 Krefeld (DE)**

EP 0 385 194 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft ein Verfahren zur Herstellung von unsymmetrischen Spiroorthocarbonaten der allgemeinen Formel I

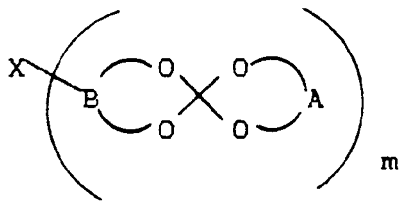

I

das dadurch gekennzeichnet ist, daß man Diaryloxyalkylendioxymethane der allgemeinen Formel II

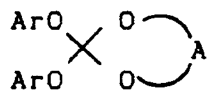

II

mit Dihydroxyverbindungen der allgemeinen Formel III

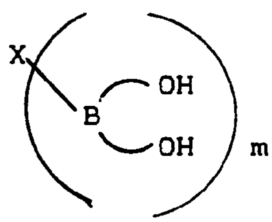

III

worin
A und B verschieden sind und gegebenenfalls substituiertes Alkylen mit 2-4 C-Atomen in der Kette darstellen,
Ar einen aromatischen Rest, bevorzugt Phenyl,
m = 1 oder 2 und
X fehlt für m = 1 und
X ein Bindeglied zwischen zwei B-Gruppen ist für m = 2 in Gegenwart von sauren Katalysatoren miteinander umsetzt.

Es ist bekannt, unsymmetrische aliphatische Spiroorthocarbonate herzustellen durch Umsetzung von Zinnglykolaten mit Schwefelkohlenstoff ausgehend von Glykolen und zinnorganischen Verbindungen (J. Org. Chem. 35, 2347 (1970). Man erhält zwar gute Ausbeuten, muß aber teure und toxische Edukte verwenden.

Deswegen wurde nach anderen Synthesemöglichkeiten gesucht und vorgeschlagen, cyclische Fünfringcarbonate in Gegenwart von Lewissäuren mit Oxiranen umzusetzen (DE-OS 3 406 049). Dies gelingt allerdings nur in unzureichenden Ausbeuten von nur wenig über 40 % an isoliertem und maximal 70 % an nachgewiesenem unsymmetrischen Spiroorthocarbonat, da in Nebenreaktionen wie Polymerbildung die Edukte verbraucht werden. Außerdem ist dieses Verfahren auf die Synthese von Fünfringverbindungen beschränkt.

Zur Synthese von unsymmetrischen Spiroorthocarbonaten mit anderen Ringgrößen geht man von Tetramethoxymethan aus, einer wenig befriedigend zugänglichen Verbindung und estert diese mit einem Diol unter Säurekatalyse zu Dimethoxy-alkylendioxymethan um, daß in 60 %iger Ausbeute isoliert wird. Dieses Orthocarbonat wird dann wiederum unter Säurekatalyse mit einem anderen Diol unter Abspaltung von Methanol umgeestert. Die Ausbeute an den gewünschten unsymmetrischen Spiroorthocarbonaten beträgt bestenfalls 44 % bezogen auf das teure Tetramethoxymethan (Synthesis 1984, 837).

Nun wurde gefunden, daß unsymmetrische Spiroorthocarbonate der Struktur I nach einem einfachen Verfahren in guter bis sehr guter Ausbeute erhalten werden, wenn man Diaryloxy-alkylendioxymethane der Struktur II mit Dihydroxyverbindungen der Struktur III in Gegenwart von sauren Katalysatoren umsetzt.

Die Reste A und B in den obigen Formeln sind jeweils verschiedene zweiwertige Alkylenreste mit 2 bis 4 C-Atomen in der Kette, die mit einem bis drei Alkylresten mit 1-10 C-Atomen, Cycloalkylresten mit drei bis acht C-Atomen, Halogenalkylgruppen und Oxyalkylgruppen mit ein bis drei C-Atomen und Chlor und Brom als Halogen, olefinischen Gruppen mit ein bis vier C-Atomen und mit romaten mit bis zu 10 C-Atomen substiutiert sein

können. Außerdem können sie eine olefinische Doppelbindung enthalten.

Allgemein kann für A und B die Formel IV angegeben werden,

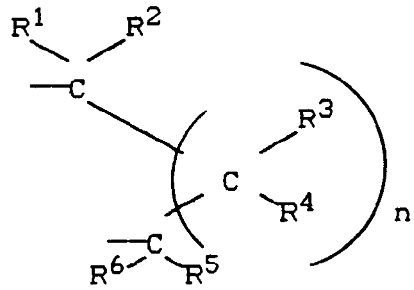

IV

worin

n eine ganze Zahl von 0 bis 2 ist,

$R^1$ bis $R^6$, H, $C_{1-10}$-Alkyl, $C_{3-8}$-Cycloalkyl, Halogenalkyl mit 1-3 C-Atomen, Oxyalkyl mit 1 - 3 C-Atomen, Phenyl, Naphthyl, Vinyl, Allyl und Methallyl,

je $R^1$ und $R^2$, $R^3$ und $R^4$, $R^5$ und $R^6$ zusammen mit dem sie verbindenden C-Atom ein 3- bis 8-gliedriges carbocyclisches Ringsystem bilden können, das auch Doppelbindungen enthalten kann, so daß spirocyclische Systeme vorliegen,

$R^2$ und $R^3$ bzw. $R^4$ und $R^5$ mit den sie verbindenden 2 C-Atomen ein 3- bis 8-gliedriges carbocyclisches Ringsystem bilden können,

außerdem für den Fall, daß n = 0 ist, die beiden verbleibenden C-Atome direkt miteinander verbunden sind und IV in IVa übergeht, $R^2$ und $R^5$ zusammen mit den nun verbundenen 2 C-Atomen einen analogen Carbocyclus bilden können,

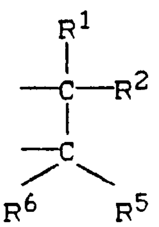

IVa

für den Fall, daß n = 1 $R^3$ und $R^4$ gemeinsam eine Methylengruppe darstellen,

und für den Fall, daß n = 2 ist, IV in IVb übergeht, die beiden mittleren C-Atome unter Elimierung jedes Substituenten $R^3$ durch eine Doppelbindung verknüpft oder Teil eines Cyclohexan- oder Benzolringes sein können, wobei die verbleibenden Substituenten $R^4$ zusammen den restlichen Teil des Cyclohexan- oder des Benzolringes darstellen,

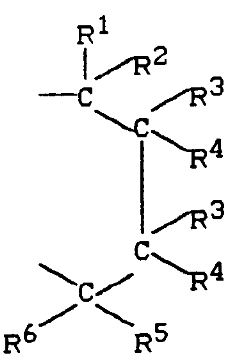

IV b

für den Fall, daß n = 1 oder 2 und m = 2 ist, einer der Reste $R^3$ oder $R^4$ das bifunktionelle Bindeglied entsprechend X in Formel I und III,

so daß die Formel III dann eine Tetrahydroxyverbindung darstellt, wobei X einfache Bindungen oder eine durch

Heteroatome unterbrochene Alkylenkette mit 2 bis 8 C-Atomen symbolisiert.

Bevorzugt ist n = 1 oder 2, und

für m = 1

$R^1$-$R^6$ gleich oder verschieden und H, $CH_3$, $C_2H_5$, Cyclopentyl, Cyclohexyl, Norbornyl, Phenyl, Chlormethyl, Allyl, Methallyl

$R^1$, $R^2$ bzw. $R^3$, $R^4$ bzw. $R^5$, $R^6$ zusammen mit dem sie jeweils verbindenden C-Atom, ein Cyclopentan-, Cyclohexan-, Cyclohexen-, Norbornen-, Norbornanringsystem,

$R^2$, $R^3$ bzw $R^4$ und $R^5$ zusammen mit den sie verbindenden 2 C-Atomen ein Cyclopentan-, Cyclohexan-, Cyclohexen-, Norbornan- oder Norbornensystem,

$R^3$ und $R^4$ gemeinsam eine Methylengruppe für n = 1,

$R^4$ und $R^4$ zusammen mit den sie verbindenden 2 C-Atomen einen Benzolring für n = 2

und weiter für n = 2 eine olefinische Gruppe für den Ausdruck in der Klammer,

für m = 2 und n = 1 ist

$R^3$ auch X und bedeutet $-CH_2OCH_2-$,

das $R^3$ und $R^4$ verbindende C-Atom X, so daß $R^3$ und $R^4$ dem zweiten B angehören und zusammen mit dem ersten B eine Spirostruktur bilden (abgeleitet z.B. von Pentaerythrit).

Die Ausgangsprodukte der Formel II und ihre Herstellung sind bekannt und in der Europäischen Patentanmeldung 190 568 beschrieben. Sie entstehen in hoher bis quantitativer Ausbeute und können häufig als Rohprodukte ohne Zwischenisolierung eingesetzt werden, so daß die unsymmetrischen Spiroorthocarbonate erfindungsgemäß im "Eintopfverfahren" hergestellt werden können. Wegen der guten Zugänglichkeit wird bevorzugt Diphenoxy-alkendioxymethan eingesetzt. Es können aber auch andere Arylgruppen verwendet werden.

Geeignete Dihydroxyverbindungen für das erfindungsgemäße Verfahren sind Ethylenglykol, Propylenglykol, Butenglykol-1,2, Cyclohexandiol-1,2, Propandiol-1,3, 2,2-Dimethylpropandiol-1,3, Cyclohexan-1,1-dimethanol, 3-Cyclohexen-1,1-dimethanol, Cyclohexan-1,2-dimethanol, Norbornan-1,2-dimethanol, 5-Norbornen-2,2-dimethanol, o-Xylylendiol, 5-Norbornen-1,2-dimethanol, Butandiol-1,3, 2-Methylbutandiol-1,3, 2-Methylbutandiol-1,4, Butandiol-1,4, Buten-2-diol-1,4, 2-Phenyl-propandiol-1,3, 2-Methylen-propandiol-1,3, 3-Chlor-propandiol-1,2, 2,2-Dichlormethylpropandiol-1,3, 2-Alkyl-hexandiol-1,3, 2,2-Diphenylpropandiol-1,3, Trimethylolpropan, Pentaerythrit, Di-n-butylether-β,β,β′,β′-tetramethanol, Oxetan-3,3-dimethanol, Thiethan-3,3-dimethanol, 1,3-Dioxan-5,5-dimethanol.

Bevorzugt sind z.B.:

Propandiol-1,3, 2,2-Dimethylpropandiol-1,3, Cyclohexan-1,1-dimethanol, 3-Cyclohexen-1,1-dimethanol, Norbornan-1,1-dimethanol, 5-Norbornen-2,2-dimethanol, 5-Norbornen-2,3-dimethanol, Butandiol-1,3, 2-Methylbutandiol-1,4, Butandiol-1,4, Buten-2-diol-1,4, 2,2-Dichlormethylpropandiol-1,3, Pentaerythrit, Di-n-butylether-β,β,β′ ,β′-tetramethanol.

Die Reaktion wird zweckmäßig in einem Lösungsmittel durchgeführt.

Geeignete Lösungsmittel sind inert unter den Reaktionsbedingungen und lösen die Reaktionspartner wenigstens zu einem geringen Teil.

Geeignete Lösungsmitel sind beispielsweise: aliphatische und aromatische Kohlenwasserstoffe wie Hexan, Heptan, Cyclohexan, Benzol, Toluol, Xylol, halogenierte Kohlenwasserstoffe wie Methylenchlorid, Chloroform, Dichlorethan, Trichlorethylen, Chlorbenzol, Dichlorbenzol, Ether wie Diethylether, Diisopropylether, Diisobutylether, Anisol, Dioxan, Methyl-tert.-butylether, Ketone wie Aceton, Methylethylketon, Diisopropylketon, Ester wie Ethylacetat, Butylacetat, Isopropylpropionat, Amide wie Dimethylformamid, Dimethylacetamid und Nitrile wie Acetonitril und Propionitril. Bevorzugt sind die halogenierten Kohlenwasserstoffe, sowie Ether und Ester.

Die Reaktion kann durchgeführt werden bei Temperaturen von -20 bis +100°C, bevorzugt -10 bis +80°C, besonders bevorzugt 0 bis 40°C. Die Reaktionszeit ist umso kürzer, je höher die Temperatur gehalten wird und reicht von einigen Minuten bis zu Stunden. Das Ende der Umsetzung läßt sich im Einzelfall leicht analytisch ermitteln.

Das Mengenverhältnis der Reaktionspartner ist an sich nicht kritisch. Es empfiehlt sich jedoch, die Reaktanden aus ökonomischen Gründen möglichst in äquimolaren Mengen einzusetzen, d.h. auf 1 Mol Diol wird ca. 1 Mol Diaryloxy-alkylendioxymethan auf 1 Mol Tetrol werden ca. 2 Mol einer Verbindung der Formel II eingesetzt.

Saure Katalysatoren im Sinne der Erfindung sind Protonensäuren mit einem $pk_a$-Wert kleiner als 5,0, bevorzugt kleiner als 4,0, besonders bevorzugt kleiner als 2,0, ganz besonders bevorzugt kleiner als 1,0. Geeignete Katalysatoren sind beispielsweise Essigsäure, Propionsäure, Benzoesäure, Phthalsäure, Chloressigsäure, Dichloressigsäure, Trichloressigsäure, die entsprechenden Fluoressigsäuren p-Nitrobenzoesäure, Dichlorbenzoesäure, Dihydroxybenzoesäure, Maleinsäure, Sulfanilsäure, Methylphosphonsäure, Phenylphosphonsäure, Toluolsulfonsäure, Naphthalinsulfonsäure, Hydroxybenzolsulfonsäure, Dichlorbenzolsulfonsäure, Benzoldisulfonsäure, saure Ionenaustauscher auf der Basis von sulfonierten vernetzten Polystyrolen oder sulfonierten Phenolharzen, Chlorwasserstoff, Salpetersäure, Schwefelsäure, Phosphorsäure, Phosphorigsäure und schweflige Säure, ferner Lewissäuren wie Aluminiumchlorid, Bortrifluorid, Bortrichlorid, Eisen-III-chlorid, Antimonpentachlorid, Titantetrachlorid, Zinntetrachlorid, Zirkontetrachlorid und Lanthantrichlorid, weiter anorganische feste Säuren wie saure Aluminiumoxide, Alumosilikate, wie säureaktivierte Schichtsilikate von Bentonit- oder Montmorillonit-Typ oder Zeolithe beispielsweise vom Typ der Pentasile oder Faujasite in der H-Form.

Die Säuren werden in katalytischen Mengen von 0,001 bis zu 50 Gew.-%, bezogen auf das Gesamtgewicht des Reaktionsmediums, bevorzugt 0,005 bis 20 Gew.-%, besonders bevorzugt 0,01 bis 10 Gw.-%, eingesetzt. Man kann feste Säuren wie Ionenaustauscher oder Zeolithe auch als Festbett anordnen und die Reaktionslösung darüber leiten.

Das Verfahren läßt sich diskontinuierlich oder kontinuierlich durchführen.

Die Aufarbeitung erfolgt in an sich bekannter Weise, indem man entweder das kristallin anfallende Spiroorthocarbonat durch Filtration abtrennt oder das gegebenenfalls vorhandene Lösungsmittel und die gebildete aromatische Hydroxyverbindung destillativ entfernt und den Rückstand der häufig schon sehr sauber ist, durch Destillation oder Umkristallisieren weiter reinigt.

<u>Beispiel</u>

Allgemeine Vorschrift

0,10 Mol Diphenoxy-alkylendioxymethan, 50 mg p-Toluolsulfonsäure und trockenes Dichlormethan werden vorgelegt und in 30 bis 60 Minuten mit 0,10 Mol einer Dihydroxyverbindung oder 0,05 Mol einer Tetrahydroxyverbindung (gegebenenfalls in Dichlormethan gelöst) tropfen- oder portionsweise versetzt. Man rührt bei Raumtemperatur, bis das Ausgangsprodukt verbraucht ist, extrahiert dann mit ca. 20 %iger Natronlauge die Säure und das entstandene Phenol aus der organischen Lösung, wäscht mit Wasser laugenfrei und destilliert das Dichlormethan ab. Das gewünschte Orthocarbonat wird im allgemeinen in reiner Form erhalten und kann durch Destillieren und Umkristallisieren weiter gereinigt werden.

| Formel | Ausbeute | m.p. |
|---|---|---|
| 1) 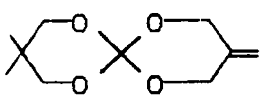 | 80 % d.Th. | 35-37° |
| aus 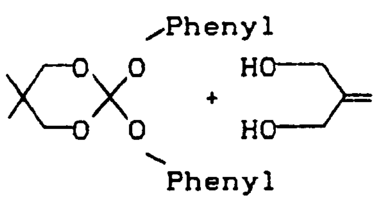 | | |
| 2) 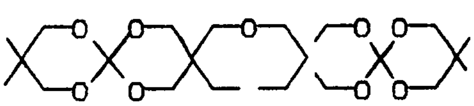 | 71 % d.Th. | 115-116° |
| aus 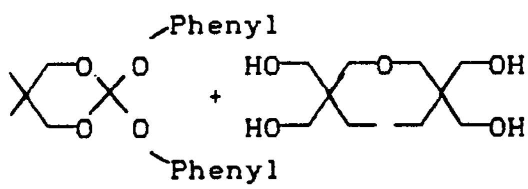 | | |

| Formel | Ausbeute | m.p. |
| --- | --- | --- |
| 3) 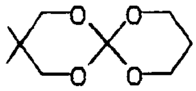 | 72 % d.Th. | 118-120$^0$ |
| aus 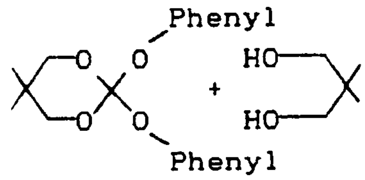 | | |

Die Ausbeuten beziehen sich auf destilliertes oder aus Ethylacetat umkristallisiertes Produkt.
Die Beispiele belegen die Flexibilität, Einfachheit und Ergiebigkeit des erfindungsgemäßen Verfahrens.

## Patentansprüche

1. Verfahren zur Herstellung von Spiroorthocarbonaten mit unsymmetrischer Struktur I

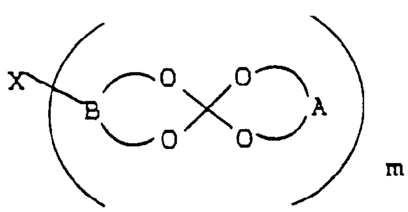

I

dadurch gekennzeichnet, daß man Diaryloxy-alkylendioxymethane der allgemeinen Struktur II

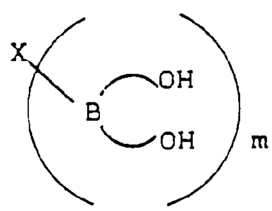

III

mit Alkandiolen der allgemeinen Struktur III

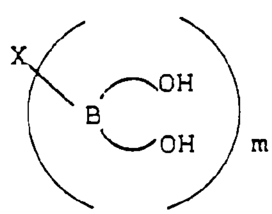

III

worin A und B verschieden sind und Alkylen mit 2-4 C-Atomen, gegebenenfalls mit einer Olefinischen Doppelbindung in der Kesse, die mit einem bis drei Alkylresten mit 1-10 C-Atomen, Cycloalkylresten mit drei bis acht C-Atomen, Halogenalkylgruppen und Oxyalkylgruppen mit ein bis drei C-Atomen und Chlor und Brom als Halogen, olefinischen Gruppen mit ein bis vier C-Atomen und mit Aromaten mit bis zu 10 C-Atomen substiutiert sein können, bedeuten,
Ar einen aromatischen Rest, m 1 oder 2, X fehlt für m = 1 und X ein Bindeglied zwischen zwei B-Gruppen ist für m = 2
in Gegenwart von sauren Katalysatoren umsetzt.

## Claims

1. Process for the preparation of spiro-orthocarbonates having the following asymmetric structure I :

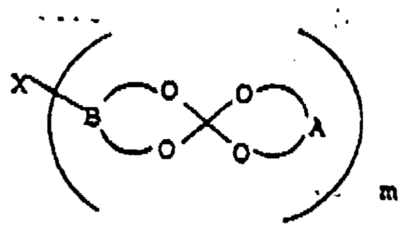

I

characterised in that diaryloxy-alkylenedioxymethanes having the following general structure II:

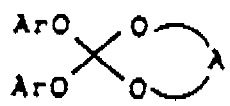

II

are reacted with alkanediols having the following general structure III:

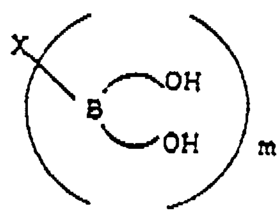

III

wherein A and B are different and denote alkylene having 2-4 carbon atoms, optionally with an olefinic double bond in the chain, which may be substituted with one to three alkyl groups having 1-10 carbon atoms, cycloalkyl groups having three to eight carbon atoms, halogenalkyl groups and oxyalkyl groups having one to three carbon atoms and chlorine and bromine as halogen, olefininc groups having one to four carbon atoms and with aromatic groups having up to 10 carbon atoms,
Ar denotes an aromatic group, m stands for 1 or 2, X is absent when m = 1 and X denotes a linking member between 2 B groups when m = 2
in the presence of acid catalysts.

## Revendications

1. Procédé de production de spiro-orthocarbonates à structure asymétrique I

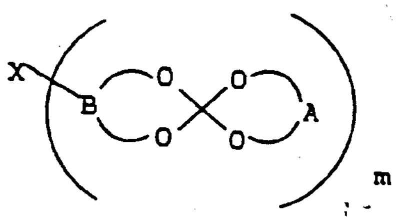

I

caractérisé en ce qu'on fait réagir en présence de catalyseurs acides, des diarylalkylènedioxyméthanes de structure générale II

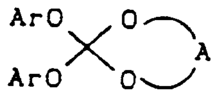

II

avec des alcane-diols de structure générale III

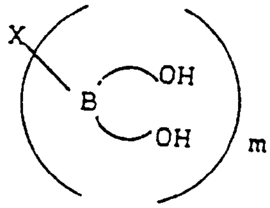

III

où A et B sont différents et représentent des groupes alkylène ayant 2 à 4 atomes de carbone, le cas échéant avec une double liaison oléfinique dans la chaîne, qui peuvent être substitués avec un à trois restes alkyle ayant 1 à 10 atomes de carbone, restes cycloalkyle ayant 3 à 8 atomes de carbone, groupes halogénalkyle et groupes oxyalkyle ayant 1 à 3 atomes de carbone et du chlore et du brome comme halogène, groupes oléfinique ayant 1 à 4 atomes de carbone et avec des groupes aromatiques ayant jusqu'à 10 atomes de carbone,
Ar est un reste aromatique, m a la valeur 1 ou 2, X est absent pour m = 1 et X est un terme de liaison entre deux groupes B pour m = 2.